(19) Europäisches Patentamt / European Patent Office / Office européen des brevets

(11) **EP 2 970 729 B1**

(12) **EUROPEAN PATENT SPECIFICATION**

(45) Date of publication and mention
of the grant of the patent:
**06.09.2023  Bulletin 2023/36**

(21) Application number: **14782366.0**

(22) Date of filing: **14.03.2014**

(51) International Patent Classification (IPC):
*C09J 191/00* (2006.01)     *A61L 24/00* (2006.01)
*A61L 24/02* (2006.01)     *A61L 24/06* (2006.01)

(52) Cooperative Patent Classification (CPC):
(C-Sets available)
**A61L 15/58; A61L 15/18; A61L 15/34; A61L 15/44;
A61L 24/0089; A61L 24/02; A61L 24/06**     (Cont.)

(86) International application number:
**PCT/US2014/028088**

(87) International publication number:
**WO 2014/168726 (16.10.2014 Gazette 2014/42)**

(54) **ADHESIVE COMPOSITION**

HAFTZUSAMMENSETZUNG

COMPOSITION ADHÉSIVE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **15.03.2013  US 201361790614 P**

(43) Date of publication of application:
**20.01.2016  Bulletin 2016/03**

(73) Proprietor: **Euromed Inc.
Orangeburg, NY 10962 (US)**

(72) Inventors:
• **RAMJIT, Ravi
Orangeburg, NY 10962 (US)**
• **GANGOLLI, Shridhar, G.
Orangeburg, NY 10962 (US)**

(74) Representative: **Haseltine Lake Kempner LLP
One Portwall Square
Portwall Lane
Bristol BS1 6BH (GB)**

(56) References cited:
**WO-A1-2011/044449     US-A- 4 505 976
US-A- 5 360 845     US-A- 6 001 345
US-A1- 2004 086 552     US-A1- 2004 241 246
US-A1- 2008 275 327     US-A1- 2011 015 619
US-A1- 2011 118 363     US-A1- 2011 130 698
US-A1- 2011 130 698     US-B1- 8 262 634**

(52) Cooperative Patent Classification (CPC): (Cont.)

C-Sets
**A61L 15/58, C08L 9/06;**
**A61L 15/58, C08L 11/00;**
**A61L 15/58, C08L 15/02;**
**A61L 24/06, C08L 53/02**

EP 2 970 729 B1

**Description**

**FIELD OF THE INVENTION**

The present invention relates to adhesive compositions applicable to skin and their use in a medical adhesive device. The adhesive compositions carry unique features, including, e.g., excellent adhesion, skin friendliness (e.g. hypoallergenicity), repositionabilty, substantially painless and residue-free detachment after application, and the ability to initiate natural antimicrobial activities.

In particular, the present invention is directed to a non-silicone based pressure sensitive hydrocolloid adhesive. The adhesive composition may be used in medical devices, particularly in skin fixation devices, wound dressing, negative pressure wound therapy (NPWT), cosmetics, transdermal delivery (TDD) and applications such as ostomy seal. The adhesive also may be used in manufacturing roll-goods, low trauma-bandages, and medical tapes in predetermined shape and size dressings. The present invention thus relates to the adhesive composition, use thereof, and to a method for the preparation of the adhesive composition.

**BACKGROUND OF INVENTION**

Pressure sensitive adhesives (PSA) medical tapes and dressings/bandages are extensively used for various wound care applications. Dressings differ from medical tapes in that they are cut to a selected size and shape and the adhesive is layered with a release liner/Z-fold. Medical tape and dressing basically consists of two major components, the adhesive and the backing material. The properties of both these components dictate the effectiveness of the finished product. The adhesive must strongly adhere to the backing material and may be applied to the backing material by spraying the adhesive solution (organic or aqueous) or by the hot- melt process. The adhesive in PSA mainly comprises polymeric material, with the principal component-base polymer being natural rubber, synthetic rubber (styrene-butadiene and ethylene copolymers, acrylic, silicones and/or ethylene-vinyl acetate (EVA) copolymers. In addition to the base polymer, the adhesive may contain other ingredients such as elastomers, rheology modifiers, fillers, water absorbents and tackifiers. The properties of the adhesive are controlled in part by varying the amount of each ingredient. The properties of pressure sensitive adhesive are controlled in part through the adhesive and backing material-tack, adhesion and cohesion.

Medical tape is primarily used to fasten medical devices and wound/surgical dressings. As with any tape products, medical tape is typically cut or ripped from a roll of a facestock or backing having at least one adhesive surface which will adhere to the skin under normal conditions without causing skin irritation, and which may be removed without any discomfort to the area of application. Medical devices may include ostomy related devices, electrodes, monitoring devices and other accessories used in the imaging or scanning process. An ideal medical tape adhesive must adhere effectively to the skin surface and devices, even on gentle application, and, during its removal process, it must not cause damage (including skin tear) or irritation to the area of application. In addition, removal of the tapes must also not leave any residue, and the tape must be removed completely without leaving any traces of adhesive on the applied area. Therefore, the adhesive properties are mainly governed by the chemical composition or matrix of the adhesive. In addition, certain properties of the finished product such as the moisture vapor transmission rate and elongation to break are also dependent on the type of backing substrate or film on which the adhesive is laminated.

Further, use of silicone based medical tapes is not possible for some patients due to its adverse skin effects or skin tolerance level.

US4505976 discloses silicone-free stoma seal adhesive compositions, comprising a pressure-sensitive adhesive component, a moisture absorbing component, and silica.

Against this background, one aspect of the invention is to provide an adhesive composition, wound dressing or ostomy product which provides good adhesion to skin, reduces the amount of pain a patient experiences upon removal and leaves no residue. Other objects of the invention will be apparent from the following description of the invention.

SUMMARY OF INVENTION

Broadly stated, the features of the invention are realized, according to one aspect of the invention, by creating an adhesive composition applicable to skin including a base polymer comprising at least copolymer selected from a specified list and silica, among other specified components. This adhesive composition carries unique features of skin friendliness, e.g. hypoallergenicity, painless removal, repositionability, and leaves no residue upon removal. The present invention relates to an adhesive composition applicable to skin comprising: (i) a base polymer comprising at least one copolymer selected from the group consisting of styrene isoprene styrene, styrene butadiene styrene, styrene ethylene-butylene styrene, styrene-ethylene-styrene, styrene-propylene-styrene, and ethylene vinyl acetate; (ii) silica; (iii) coconut oil; (iv) a mineral oil; (v) a tackifier selected from the group consisting of natural rosin, modified rosin, glycerol ester of

3

natural rosin, glycerol ester of modified rosin, pentaerythritol ester of natural rosin, pentaerythritol ester of modified rosin, phenolic-modified terpene resin, aliphatic petroleum hydrocarbon resin, and cycloaliphatic resin; and (vi) a gel-thickener selected from the group consisting of carboxymethylcellulose (CMC), hydroxyethylcellulose (HEC), hydroxypropylcellulose (HPC), methylcellulose, hydroxypropylmethylcellulose, sodium starch glycolate, polyvinyl alcohol, polyethylene glycol, pectin, carrageenan, and gelatin; wherein the composition is silicone-free; and wherein the ratio of (a) base polymer to coconut oil ranges from 1:1 to 1.5:1; (b) base polymer to mineral oil ranges from 0.5:1 to 0.6:1; (c) base polymer to tackifier ranges from 1.3:1 to 1.7:1; (d) base polymer to silica ranges from 38:1 to 78:1; (e) base polymer to gel-thickener ranges from 0.7:1 to 2.5:1; (f) mineral oil to coconut oil ranges from 2:1 to 2.5:1.

The present invention relates to an adhesive composition that is silicone-free.

The present invention relates to an adhesive composition which includes coconut oil.

In one embodiment, the present invention relates to a medical adhesive device including the above adhesive composition, wherein the amount of the coconut oil and mineral oil is in the range of about 48%-60% (w/w) of the total composition.

Also described herein is a method of preparing the adhesive (1) by the hot melt process wherein individual components are blended and mixed in a heated mixer or (2) by dissolving and stirring the individual components in a suitable organic solvent. Finished product from the adhesive obtained from method (1) is obtained by pressing the adhesive between two liners or films, while that from method (2) is obtained by casting the adhesive solution onto the desired film or liner and evaporating the solvent to obtain a thin adhesive film.

**DESCRIPTION OF DRAWINGS**

Figure 1 is a rheology thermogram showing the temperature ramp plot of shear modulus G'[Pa] vs. temperature for adhesives prepared with and without silica.

Figure 2 is a rheology thermogram showing the frequency sweep plot of loss tangent (tan $\delta$) vs. angular frequency obtained at 37°C for adhesives prepared with and without silica.

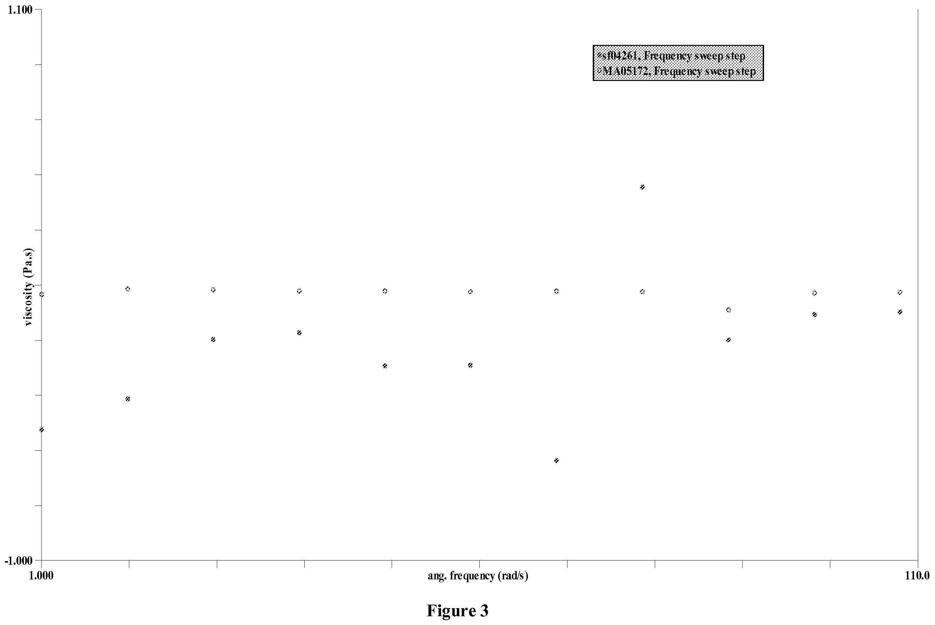

Figure 3 is a rheology thermogram showing the frequency sweep plot of viscosity vs. angular frequency at 37°C for adhesives prepared with and without silica.

Figure 4 is a rheology thermogram showing the effect of silica on rheology of the adhesive.

**DETAILED DESCRIPTION OF THE INVENTION**

The present invention relates to adhesive compositions that maybe used for medical devices, particularly, for skin fixation devices, wound dressings, negative pressure wound therapy (NPWT), cosmetics, transdermal delivery (TDD) and in ostomy fixation.

The pressure sensitive adhesive composition of the present invention differs from previous compositions in that the previous compositions do not include silica. In addition to easy removal of the tape, the use of silica, for example, allows the composition to maintain its moisture content. The ease of removal is not significantly affected by the particle of the silica.

The pressure sensitive adhesive composition of the present invention differs from previous compositions in that the previous compositions do not include reinforcing agents. Such reinforcing agents are used in part to ease the tape removal process in addition to maintaining wound moisture level.

It has been found that the pressure sensitive adhesive compositions with silica and other components exhibit unique features that may be used for medical devices, particularly, for skin fixation devices, wound dressing, negative pressure wound therapy (NPWT), cosmetics, transdermal delivery (TDD) and applications such as ostomy seal. Features such as good adhesion to skin, reasonable water absorption, low hydrocolloid thickness, and easy removal without leaving behind any residue, are obtained by varying the ratio of different components or raw materials. The adhesive is also useful under very fragile and sensitive skin conditions.

In one embodiment, the low trauma or skin friendly adhesive is a complex blend including an elastomer, a tackifier, a botanical triglyceride of fatty acids (coconut oil), an inorganic filler (silica)/binder (e.g., cross-linked polyacrylates), a plasticizer/dispersing medium (mineral oil), a reinforcing agent and water absorbent components. The order of addition of each component is critical in determining the bulk properties and the manufacturing cost of the hydrocolloid adhesive.

Each component used in the adhesive plays a definite role in improving the properties of the adhesive. The botanical coconut oil in the adhesive, in addition to acting as a plasticizer, acts as a penetration enhancer, thus providing for the incorporation of active pharmaceutical ingredients into the adhesive, in which case the pressure sensitive tape/adhesive maybe used in a transdermal delivery system. Mineral oil may serve both as a plasticizer and a solvent for the elastomer. The elastomer may be a combination of two components. The use of multiple elastomers may induce desirable properties in the adhesive and reduce the cost of the final finished product. The type and nature of the tackifier influences the tack property of the adhesive. The inorganic filler (silica) plays an important role in the trauma- free removal of the dressing without causing tearing or damage to the patient skin in addition to maintaining partial moisture content. The moisture absorption capacity of the adhesive may be controlled by the amount and type of moisture absorbing component in the adhesive matrix. The amount of moisture in this component/raw material also determines the moisture absorbing capacity of the adhesive. Use of reinforcing agent in the adhesive improves the smooth removal of the finished product. Due to the viscous adhesive character, this formulation has a unique feature whereby dressings maybe made with low hydrocolloid thickness.

In this present invention, the design approach is directed toward obtaining an adhesive that may be easily removed but has reasonable skin adhesion, prevents skin tear, and absorbs and controls moisture levels. Due to the soft characteristics of the adhesive, it may be laminated or coated on a backing material to any desirable thickness, usually less than 0.5mm, and may incorporate an active pharmaceutical ingredient.

In one embodiment of the present invention, the adhesive composition comprises various types of silica, at least one reinforcing agent and at least one homopolymer and /or block copolymer. The homopolymer may include, but is not limited to, isobutylene and polystyrene.

In one embodiment, the homopolymer is a homopolymer rubber. Examples of homopolymer rubber include, but are not limited to, isobutylene, epichlorhydrin rubber, chloroprene rubber, isoprene rubber, bromobutyl rubber, and chlorobutyal rubber.

A suitable copolymer may contain at least two monomers which have very different glass transition temperatures so that they are immiscible in each other and phase separate at room temperature. Examples of such copolymers include, but are not limited to, styrene isoprene styrene (SIS), styrene butadiene styrene (SBS), styrene ethylene-butylene styrene (SEBS), styrene-ethylene-styrene (SES), styrene-propylene-styrene (SPS), and ethylene vinyl acetate (EVA). In one embodiment, the copolymer is selected from the group consisting of Kraton™ D 1113, Kraton™ D 1173, Kraton™ D 1107 (Shell Chemicals), Kraton™ D 1100, Kraton™ D1102, Kraton™ 4000, Kraton™ G1600, Kraton™ G4600 and mixtures thereof. In one embodiment, the copolymer is an SIS polymer such as Kraton™ D1107 (Shell Chemicals).

In one embodiment, the copolymer is SIS. SIS may form the base polymer that acts as the foundation to build the adhesive. The styrene and the isoprene have very different glass transition temperatures (Tg) (+100°C and -60°C respectfully). They are immiscible and phase separate at room temperature: the styrene crystallizes, while the isoprene remains as a liquid. Since SIS has styrene on both ends of the isoprene chains, the hardened styrene crystals act as nanoscopic physical cross-linkers and provide integrity to the adhesive, which gives the adhesive mix its cohesive strength. Isoprene, on the other hand, has low Tg, and its Tg has to be modified by means of additional tackifier. In addition, the G' of the adhesive may be modified by addition of plasticizer, often oil or fat. Depending on the chemistry of the chosen oil and the tackifier, they may have various degrees of affinity toward styrene and isoprene. In general, most oils and tackifiers are chosen to have good affinity to the mid-block isoprene domain (I) and moderate to no affinity to the end-block styrene domains (S) to preserve cohesive strength.

Suitable tackifiers often have low molecular weight with higher Tg than the isoprene. Tackifiers are selected from natural rosin, modified rosin, glycerol ester of natural rosin, glycerol ester of modified rosin, pentaerythritol ester of natural rosin, pentaerythritol ester of modified rosin, phenolic-modified terpene resin, aliphatic petroleum hydrocarbon resin, and cycloaliphatic resin.

The present adhesive compositions comprise fluid absorbent materials which are classified as hydrocolloids (HC). Hydrocolloids are used in skin fixation devices, wound dressings, negative pressure wound therapy (NPWT), cosmetics, transdermal patches, and in ostomy applications. In skin fixation and wound dressing applications, the hydrocolloid adhesive mix is laminated to a polymer film to form an adhesive laminate. This laminate maybe applied to intact skin (skin fixation) or wounds (wound dressing), acting as an environmental barrier and a wound cushion, and taking up excess body sweat and wound exudate. In ostomy fixation system, pure hydrocolloid adhesive is pressed into a ring shape and is used to bind the ostomy pouch onto the skin. In addition, the adhesive may be used as a protective barrier or sealant around the stoma to block the effluent from contacting peri-stoma skin.

The present compositions maybe applied to medical fixation, such as IV dressing, adhesive foam, and bordered foam dressings. Such medical fixation will carry the features of painless removal and avoidance of damage to periwound skin. The IV dressing is a transparent or translucent thin dressing that acts as a skin barrier to cover the IV injection port. The adhesive foam is a foam dressing with an adhesive coating on the skin contact side. The adhesive allows the foam to adhere and provides a gentle bond to the wound site without the need for a nurse to apply secondary adhesive to frame the foam border. The bordered foam dressing has the adhesive border pre-laminated to the foam, and the dressing is used as is, without the need for secondary adhesive.

Suitable hydrophilic fluid-absorbing gum or gel-thickener serves the dual purpose of taking up moisture and providing extra cohesive strength for the adhesive composition. The suitable hydrophilic fluid-absorbing gum or gel-thickener provides gentle tack and does not provide fluid absorption.

As used herein, the term "benefit agent" includes any active ingredient that is to be delivered into and/or onto the skin, hair or nail at a desired location, such as a cosmetic agent or a pharmaceutical agent.

By "cosmetic agent," it is meant any ingredient that is appropriate for cosmetically treating, providing nutrients to, and/or conditioning, e.g., the hair, nail, and/or skin via topical application.

By "pharmaceutical agent," it is meant any drug that is either hydrophobic or hydrophilic in nature and appropriate for topical use.

Examples of suitable benefit agents include, but are not limited to, analgesics, anti-inflammatory agents, both of steroidal and non-steroidal nature, antihistamines, antipruritics, general and local anesthetics, vasoconstrictors, antihypertensives including vasodilators, diuretics and ACE inhibitors, cardiac agents, hemostatics and styptics, mucolytics, antitussives, expectorants, mucoprotectants, antineoplastics, immunologic agents, antibiotics, antivirals, antidiabetics, bronchodilators, sympathomimetics, adrenergics, adrenergic blockers, anticholinergics, antimuscarinics, antispasmodics, skeletal muscle relaxants, uterine and antimigraine drugs, sedatives, hypnotics, anxiolytics, central nervous system stimulants, antidepressants and other psychopharmaceutical agents, antiepileptics, antiemetics and hormones.

Analgesics include, but are not limited to, opiate and non-opiate analgesics and antagonists of both synthetic and natural origin. Examples include, but are not limited to, morphine derivatives, codeine derivatives, methadone, propoxyphene, meperidine, fentanyl, morphinans such as levorphanol, and pentazocine. Other analgesics include, but are not limited to, acetaminophen.

Some examples of non-steroidal anti-inflammatory agents include, but are not limited to, propionic acids such as fenoprofen, ibuprofen, ketoprofen; fenamates such as meclofenamate and mefenamic acid; acetic acids such as diclofenac, etodolac, indomethacin, sulindac; oxicams such as piroxicam; and other agents such as nabumetone and oxyphenbutazone. Additionally, the following agents are also known as analgesic/anti-inflammatory agents: salicylates such as aspirin, methyl salicylate and monoglycol salicylate; salsalate; gold compounds such as auranofm; allopurinol; colchicine; and methysergide.

Examples of steroidal anti-inflammatory agents include, but are not limited to, hydrocortisone, prednisolone, dexamethasone, triamcinolone, fluocinolone, methylprednisolone, betamethasone, flumetasone, fluorometholone, beclomethasone and fluocinonide.

Antihistamines may be of $H_1$ or $H_2$ antagonists or other types of histamine release inhibitors. The $H_1$ antagonists may be sedating or non-sedating. Examples of $H_1$-sedating antihistamines include, but are not limited to, diphenhydramine, chlorpheniramine, tripelennamine, promethazine, clemastine and doxylamine. Examples of $H_1$-non-sedating antihistamines include, but are not limited to, astemizole, terfenadine and loratadine. Examples of $H_2$ antagonists include, but are not limited to, cimetadine, famotidine, nizatidine, and ranitidine. An example of a histamine-release-inhibitor is cromolyn.

Examples of local anesthetics include, but are not limited to, dibucaine, lidocaine, benzocaine, p-butylaminobenzoic acid-2-(diethylamino) ethyl ester, procaine, tetracaine, chloroprocaine, oxyprocaine, mepivacaine, bupivacaine, cocaine, piperocaine, and dyclonine. Examples of vasoconstrictors include, but are not limited to, naphazoline, tetrahydrozoline, oxymetazoline and phenylephrine.

Examples of hemostatics and styptics include, but are not limited to, thrombin, phytonadione, protamine, aminocaproic acid, tranexamic acid, rutin, hesperidin, silver salts, and ferric salts.

Examples of antibacterials include, but are not limited to, sulfa drugs, penicillins, cephalosporins, tetracyclines, erythromycins, aminoglycosides, polypeptide antibiotics, fluoroquinolones, chloramphenicol, clindamycin, rifampin, spectinomycin, vancomycin, bacitracin, cyclosporine, dapsone, ethambutol, ethionamide, isoniazid, nitrofurantoin, pyrazinamide, and trimethoprim. Additional agents include antimalarials, amebicides, antiprotozoals, anthelmintics, pediculicides and scabicides.

Examples of antiviral drugs include, but are not limited to, viral DNA polymerase inhibitors such as foscarnet, protease inhibitors, thymidine kinase inhibitors, sugar or glycoprotein synthesis inhibitors, structural protein synthesis inhibitors, attachment and adsorption inhibitors, amantadine, and nucleoside analogues such as acyclovir, didanosine, ganciclovir, idoxuridine, ribavarin, trifluridine, vidarabine, zalcitabine, zidovudine, acyclovir, penciclovir, valacyclovir, and ganciclovir.

Examples of mucolytics include, but are not limited to, potassium iodide, sodium thiocyanate, urea, guanidine hydrochloride, N-acetylcysteine, dithiotheritol, and proteolytic enzymes such as chymotrypsin and trypsin. These agents may be used to affect mucus production and the elasticity and viscosity of the mucus produced.

Examples of hormones include, but are not limited to, insulin, LHRH, growth hormone, calcitonin, thyroid hormones, and male and female hormones such as testosterones, estrogens and progesterones.

Examples of astringents include, but are not limited to, aluminum salts such as alum, aluminum acetate, aluminum chloride, aluminum chlorohydrates, aluminum sulfate, aluminum zirconium chlorohydrate, bismuth subcarbonate, bismuth subnitrate, calamine, glutaral, methenamine, potassium permanganate, resorcinol, silver nitrate, tannic acid, zinc caprylate, zinc chloride, zinc oxide, zinc pyrithione, zinc sulfate and zinc undecylenate.

Examples of irritants, rubifacients, and vesicants include, but are not limited to, anthralin, benzoin tincture, camphor, cantharidin, capsicum, coal tar, ichthammol, juniper tar, menthol, balsams such as Peruvian balsam and Tolu balsam.

Topical antifungals include, but are not limited to, haloprogin, ciclopirox, flucytosine, miconazole, econazole, clotrimazole, fluconazole, oxiconazole, sulconazole, metronidazole, itraconazole, ketoconazole, butaconazole, terconazole, nystatin, povidone-iodine, tolnaftate, benzoic acid, salicylic acid, mercuric oxide, resorcinol, triacetin, undecylenic acid and its calcium, copper and zinc salts.

Topical anesthetics include, but are not limited to, the local anesthetics described above and benzyl alcohol, camphor, camphorated metacresol, juniper tar, menthol, phenol, phenolate sodium, resorcinol, methyl salicylate, turpentine oil, camphor, menthol, methyl nicotinate, capasaicin, capsicum containing capsaicin, and capsicum oleoresin containing capsaicin.

Examples of keratolytics and cauterizing agents include, but are not limited to, salicylic acid, podophyllum resin, podolifox, cantharidin, the chloroacetic acids and silver nitrate.

Examples of topical bactericides and disinfectants include, but are not limited to, thimerosal, phenol, thymol, benzalkonium chloride, benzethonium chloride, chlorhexidine, povidone iodine, cetylpyridinium chloride, eugenol, and trimethylammonium bromide.

Short-chain fatty acids (SCFA) are fatty acids with aliphatic tails of fewer than six carbons. Short chain fatty acids useful in the practice of the present invention include, but are not limited to, acetic acid, propionic acid, isobutyric acid (2-methylpropanoic acid), butyric acid, isovaleric acid (3-methylbutanoic acid), valeric acid (pentanoic acid), and caproic acid (hexanoic acid).

Medium-chain fatty acids (MCFA) are fatty acids with aliphatic tails of 6-12 carbons. Medium chain fatty acids useful in the practice of the present invention include, but are not limited to, caproic acid (C6), caprylic acid (C8), capric acid (C10) and lauric acid (C12). In an embodiment of the invention, coconut oil, which is a blend of 2(C6):55(C8):42(C10):1(C12), is a source of fatty acids.

Long-chain fatty acids (LCFA) are fatty acids with aliphatic tails longer than 12 carbons. Long chain fatty acids useful in the practice of the present invention include, but are not limited to, myristic acid (14 carbons), palmitic acid (16 carbons), oleic acid (18 carbons - monounsaturated), stearic acid (18 carbons - saturated) and erucic acid (22 carbons). In one embodiment of the invention, myristic acid and palmitic acid are preferred long-chain fatty acids.

Very-long-chain fatty acids (VLCFA) are fatty acids with aliphatic tails longer than 22 carbons. Very-long-chain fatty acids may be useful in the adhesive compostions of the present invention.

Essential Fatty Acids useful in the practice of the present invention include, but are not limited to, alpha linolenic acid, eicosapentaenoic acid, docosahexaenoic acid, and gamma linolenic acid. In an embodiment of the invention, alpha linolenic acid and eicosapentaenoic acid are components of the adhesive compositions of the present invention.

To obtain excellent uniformity in the adhesive, fillers or reinforcing agents with high surface area are used as components in the adhesive compositions of the present invention.

Suitable hydrophilic liquid absorbers or gel-thickeners are selected from naturally occurring hydrocolloids, semisynthetic hydrocolloids and synthetic hydrocolloids.

Hydrocolloids useful in the practice of the present invention include, but are not limited to, water absorbing and/or water swellable material such as carboxymethylcellulose (CMC), hydroxyethylcellulose (HEC), hydroxypropylcellulose (HPC), methylcellulose, hydroxypropylmethylcellulose, sodium starch glycolate, polyvinyl alcohol, polyethylene glycol, pectin, gelatin, high molecular weight carbowax, carboxypolymethylene, carboxymethyl starches, alginates, carrageenan, gelatin, citrus pectin, powdered pectin, synthetic or natural gums, such as gum guar, gum arabic, locust bean gum, karaya and mixtures thereof.

In an embodiment of the invention, the wound dressing may contain elastomeric binders and tackifiers.

Elastomeric binders useful in the practice of the present invention include, but are not limited to, diblock, triblock, or multiblock elastomeric copolymers such as olefinic copolymers (e.g., styrene-isoprene-styrene, styrene-butadiene-styrene, styrene-ethylene/butylene-styrene, and styrene-ethylene/propylene-styrene). Exemplary elastomeric binders include those available from the Shell Chemical Company, under the trade designation KRATON® elastomeric resin; polyurethanes, such as those available from E. I. Du Pont de Nemours Co., under the trade name LYCRA® polyurethane; polyamides, such as polyether block amides available from Ato Chemical Company, under the trade name PEBAX® polyether block amide; or polyesters, such as those available from E. I. Du Pont de Nemours Co., under the trade name HYTREL® polyester; natural rubbers, silicone rubber, polyisobutylene rubber, and acrylonitrile rubber. In an exemplary embodiment, the KRATON® olefinic copolymers are used in the adhesive compositions of the claimed invention.

A tackifier is used to control the tack of the adhesive and reduce moduli and increase glass transition temperature. Tackifiers according to the invention are natural rosin, modified rosin, glycerol ester of natural rosin, glycerol-ester of modified rosin, pentaerythritol ester of natural rosin, pentaerythritol ester of modified rosin, phenolic modified terpene resin, aliphatic petroleum hydrocarbon resin, and cycloaliphatic resin. Representative tackifiers, known by their tradenames are Kristalex™ 3085, FORAL® 85 and ARKON® P115. Kristalex™ 3085 and FORAL® 85 are hydrocarbon tackifiers. ARKON® PI 15 is a hydrogenated hydrocarbon tackifier. In an embodiment of the invention, Kristalex™ 3085 is used as tackifier.

In one of the embodiment of the present invention, the adhesive composition comprises botanical penetration enhancer. The botanical penetration enhancer is coconut oil. The coconut oil may be dehydrogenated or hydrogenated.

In one embodiment of the present invention, the adhesive composition comprises silica and at least one copolymer, a botanical penetration enhancer, a tackifier and a gel-thickener.

In one of embodiment of the present invention, the adhesive composition comprises silica and at least one copolymer, a tackifier, a filler, an inhibitor, and one or more stabilizers.

In one of embodiment, the present invention relates to an adhesive composition comprising a mineral oil and optionally a plasticizer selected from the group consisting of citrate oil, paraffin oil, phthalic acid esters, adipic acid esters, liquid or solid resins, and mixtures thereof.

In another embodiment of the present invention, the adhesive composition comprises other ingredients, including, e.g., antioxidants, stabilizers, fillers, pigments, flow modifiers, and active ingredients such as drugs. The filler or reinforcing material may be silica, which is treated or non-treated, complexed or chelated.

In one embodiment, the present invention relates to a pressure sensitive adhesive composition for skin application comprising 15-25% (w/w) based on the total mixture formulation of a blend of at least one homopolymer and /or copolymer, e.g. polystyrene - isoprene-polystyrene or polystyrene- isoprene-butadiene and silica/polyacrylate.

In one embodiment, the present invention relates to a pressure sensitive adhesive composition wherein the blend of copolymer and silica is less than 15% (w/w) of the total mixture formulation, and the content of the homopolymer and copolymer is less than 30% (w/w) of the total mixture formulation.

In one embodiment, the present invention relates to a pressure sensitive adhesive composition for skin application comprising 15-25% (w/w) based on the total mixture formulation of a blend of at least one homopolymer and botanical oil.

In one embodiment, the present invention relates to a pressure sensitive adhesive composition for skin application comprising 50% of botanical oil based on the plasticizer content.

In one embodiment, the final adhesive in continuous form exhibits a water absorption rate of 13%/24 hours at 37°C.

In one embodiment, the final adhesive has a complex modulus G' of less than 20000 Pa. In one embodiment, the final adhesive has a complex modulus G' of less than 30000 Pa at 1 Hz (1% deformation, 32°C).

In one embodiment, the final adhesive has a tan(δ) above 0.2. In one embodiment, the final adhesive has a tan(δ) above 1.6 at 1Hz ( 1% deformation, 32°C).

In one embodiment, the content of the adhesive blend comprises a tackifier resin such as natural, modified or synthetic resins.

In one embodiment, the content of the tackifying resin is from 0-25% (w/w) of the final adhesive. In one embodiment, the content of the tackifying resin is from 5-20% (w/w) of the final adhesive. In one embodiment, the content of the tackifying resin is from 7-15% (w/w) of the final adhesive.

In one embodiment, the composition further comprises other ingredients, including, e.g., stabilizers, fillers, pigments, flow modifiers and active ingredients.

In one embodiment, the composition further comprises one or more absorbing particles such as a cellulose hydrocolloid.

In one embodiment, the content of cellulose hydrocolloid is about 5-25% (w/w) of the total composition.

In one embodiment, the composition further comprises one or more reinforcing agent and water absorption modifiers.

In one embodiment, the present invention relates to a low trauma tape comprising a pressure sensitive adhesive composition comprising a blend of at least one homopolymer and /or copolymer and one or more silica/polyacrylate compounds.

In one embodiment, the medical tape may be used for skin fixation applications, such as catheter fixation, medical devices, and ostomy seals.

The invention is illustrated more in detail in the below examples disclosing embodiments of the invention.

EXAMPLES

In one embodiment, the adhesive compositions of the present invention are made according to the following methods.

An adhesive composition by the hot melt process was prepared by adding the base polymer into a heated mixer in which the temperature in the mixer was set at 180°C and the temperature measured inside the mixer was between 110-120°C. The base polymer was allowed to tumble in the mixer between 5-15 minutes or until the base polymer showed coarse particles. The plasticizer was then added in small aliquots to obtain a uniform smooth mass. The mix was allowed to tumble for 5-15 minutes. The set temperature was then reduced so that the temperature inside the mixer was between 100-110°C. At this point, small amounts of the penetration enhancer or botanical component were added. To reduce the processing time, it was necessary to add the plasticizer before adding the penetration enhancer. At this point, the filler component of the mix was added, followed by the tackifier and the water absorbent. The temperature inside the mixer was reduced to between 90-100°C before the addition of the water absorbent. To obtain content uniformity in the adhesive, the mix was allowed to tumble for at least 10 minutes before adding the next component. At the end of the mixing process, the adhesive was poured and allowed to cool at room temperature before manufacturing the finished product.

An alternative method to prepare the adhesive is by dissolving each component of the adhesive in an organic solvent such as toluene or heptane. In order to achieve complete solubility of each component, the mix solution was stirred at room temperature for about 24 hours. The stirring time may be reduced if the stirring was performed at higher temperature. At the end of the mixing period, the adhesive solution is cast on the desired film and the solvent is then evaporated to obtain an adhesive of desired thickness. **Example 1:** Representative components used in the adhesive composition of the present invention.

**Table 1**

| Material | Manufacturer or Supplier | Representative Property |
|---|---|---|
| Kraton 1113, Kraton 1173 | Kraton Polymers US | Base Polymer |
| Mineral Oil | Sonneborn Inc | Plasticizer |
| Hydrogenated Coconut Oil | Welch, Holme & Clark Co | Penetration Enhancer |
| Aerosil 200 | Evonik Industries | Filler |
| Kristalex 3085 | Eastman | Tackifier |
| Sodium carboxymethylcellulose | Amtex Chemicals | Water Absorbent |

**Example 2:** Representative composition according to one aspect of the present invention.

**Table 2**

| Description | MA05172 % by wt. | MA09211* % by wt. |
|---|---|---|
| Kraton 1113 | 19.95 | 21.16 |
| Mineral Oil | 39.90 | 34.39 |
| Hydrogenated Coconut Oil | 19.95 | 13.76 |
| Aerosil 200 | 0.53 | 0.27 |
| Kristalex 3085 | 11.70 | 15.87 |
| CMC- Amtex | 7.98 | 14.55 |
| Total | 100.00 | 100.00 |
| *MA09211 is equivalent to sf07272 | | |

**Example 3:** Representative composition according to one aspect of the present invention. sf04261 has same composition as MA05172 but without Aerosil 200.

**Table 3**

| Description | MA05172 % by wt. | sf04261 % by wt. |
|---|---|---|
| Kraton 1113 | 19.95 | 20.05 |
| Mineral Oil | 39.90 | 40.11 |
| Hydrogenated Coconut Oil | 19.95 | 20.05 |
| Aerosil 200 | 0.53 | - |
| Kristalex 3085 | 11.70 | 11.76 |

(continued)

| Description | MA05172 % by wt. | sf04261 % by wt. |
|---|---|---|
| CMC- Amtex | 7.98 | 8.02 |
| Total | 100.00 | 100.00 |

**Example 4:** Representative compositions according to one aspect of the present invention. Adhesive compositions were prepared with different type of silica or filler.

**Table 4**

| Description | sf07311 | sf10051 | sf10052 | sf10081 | sf02041 |
|---|---|---|---|---|---|
| Kraton 1113 | 21.69 | 20.20 | 20.20 | 20.20 | 27.59 |
| Mineral Oil | 36.88 | 32.83 | 32.83 | 32.83 | 22.41 |
| Hydrogenated Coconut Oil | 11.93 | 13.13 | 13.13 | 13.13 | 17.93 |
| Filler/Reinforcing Agent | 0.22 (S974) | 1.01 (S500) | 1.01 (OX50) | 1.01 (S22LS) | 1.03 (S200) |
| Kristalex 3085 | 16.27 | 15.15 | 15.15 | 15.15 | 13.79 |
| CMC- Amtex | 13.02 | 17.68 | 17.68 | 17.68 | 17.24 |
| Total | 100 | 100 | 100 | 100 | 100 |

**Example 5:** Representative compositions according to one aspect of the present invention The adhesive compositions were prepared with the same composition as MA05172 but with an Styrene-Isoprene-Butadiene-Styrene (SIBS) base polymer and mixture of copolymers.

**Table 5**

| Description | MA05172 % by wt. | sf05181 % by wt. | sf04131 % by wt. |
|---|---|---|---|
| Kraton 1113 | 19.95 | - | 14.42 |
| Kraton 1173 | - | 19.95 | 14.42 |
| Mineral Oil | 39.90 | 39.90 | 41.54 |
| Hydrogenated Coconut Oil | 19.95 | 19.95 | 10.00 |
| Aerosil 200 | 0.53 | 0.53 | 0.38 |
| Kristalex 3085 | 11.70 | 11.70 | - |
| CMC- Amtex | 7.98 | 7.98 | 19.23 |
| Total | 100.00 | 100.00 | 99.99 |

**Example 6:** Representative compositions according to one aspect of the present invention. The adhesive compositions were prepared with different fillers/water absorbing species concentration. These compositions were characterized for water absorbing property.

**Table 6**

| | Raw Material (%) | | | | | | |
|---|---|---|---|---|---|---|---|
| Composition ID | Kraton | Mineral Oil | Coconut Oil | Aerosil S500 | Kristalex 3085 | CMC | Inhibitor |
| sf10122 | 19.80 | 32.18 | 12.87 | 2.97 | 14.85 | 17.33 | - |
| sf10231 | 26.60 | 44.33 | 21.28 | 4.26 | | 3.55 | - |
| sf10232 | 26.42 | 43.89 | 21.05 | 9.40 | 0.90 | 3.54 | - |
| sf10233 * | 26.63 | 44.88 | 21.54 | 4.31 | - | 1.80 | 0.54 |

(continued)

| Composition ID | Raw Material (%) | | | | | | |
|---|---|---|---|---|---|---|---|
| | Kraton | Mineral Oil | Coconut Oil | Aerosil S500 | Kristalex 3085 | CMC | Inhibitor |
| sf10261 | 20.62 | 39.69 | 13.40 | 3.09 | 5.15 | 18.04 | - |
| *Kraton K1173 | | | | | | | |

**Example 7: Testing of composition by water absorption measurement**

A. Sample pressing conditions and procedure:

A small portion of the adhesive was pressed between a clear silicone liner by compressor mold at 85°C and 8000lb force, dwell time 8 sec with thickness of 1 mm. The sample was prepared by punching a 1' diameter circle, and water absorption was determined as per Euromed Inc method.

B. Method to determine water absorption

The sample with the clear silicon liner was weighed. The clear silicon liner was removed and weighed. The weight of the sample was then determined ($W_0$). The sample was then immersed in a tray well containing 0.9% saline at 37°C oven. The sample was weighed at the desired time ($W_t$) and the % of water absorbed was determined as shown below.

$$\% \text{ of water absorbed} = ([W_t - W_0]/W_0) * 100$$

C. Results

7.1. Water Absorption for adhesive composition at 3 time points

Table 7.1

| Composition ID | Water Absorption (%) at | | |
|---|---|---|---|
| | 3.5h | 16h | 24h |
| sf10U51 | 252 | 558 | 355 |
| sf10052 | 73 | 539 | 424 |
| sf10081 | 146 | 62 | 411 |

7.2. Water Absorption for adhesive composition at 2 time points

Table 7.2

| Composition ID | Water Absorption (%) at | |
|---|---|---|
| | 6h | 24h |
| sf10121A | 211 | 633 |
| sf10121B | 547 | 580 |
| sfl0122 | 580 | 672 |
| sf10221 | 8 | 9 |

(continued)

| Composition ID | Water Absorption (%) at | |
| --- | --- | --- |
| | 6h | 24h |
| sf10231 | 4 | 6 |
| sf10232 | 3 | 6 |
| sf10233 | 5 | 9 |

7.3. Water Absorption for adhesive composition at 6 time points

**Table 7.3**

| Composition ID | Water Absorption (%) at | | | | | | |
| --- | --- | --- | --- | --- | --- | --- | --- |
| | 1h | 2h | 4h | 6h | 16h | 24h | 30h |
| sf10261 | 34 | 47 | 49 | 73 | 466 | 424 | 461 |
| sf11121 | 34 | 78 | 160 | 351 | 648 | 607 | 642 |
| sf07272 | 6 | 5 | 9 | 9 | 195 | 196 | 277 |
| sf04261 | 7 | 8 | 7 | 10 | 11 | 13 | - |
| sf02041 | 18 | 35 | 36 | 62 | 373 | 383 | - |
| sf05181 | 3 | 3 | 5 | 6 | 8 | 14 | - |
| MA05172 | 3 | 4 | 8 | 7 | 13 | 19 | - |

**Example 8: Clinical Example**

The wound dressing composition of the present invention is effective in the management of the following exemplary conditions:

i. Chronic and acute, moderate to heavy exudating, partial and full thickness wounds including superficial wounds
ii. 2nd degree burns
iii. Pressure ulcers, Stages II -IV

**Example 9: Significance of Silica in the adhesive composition**

a) Comparison of Shear Modulus G'[Pa]

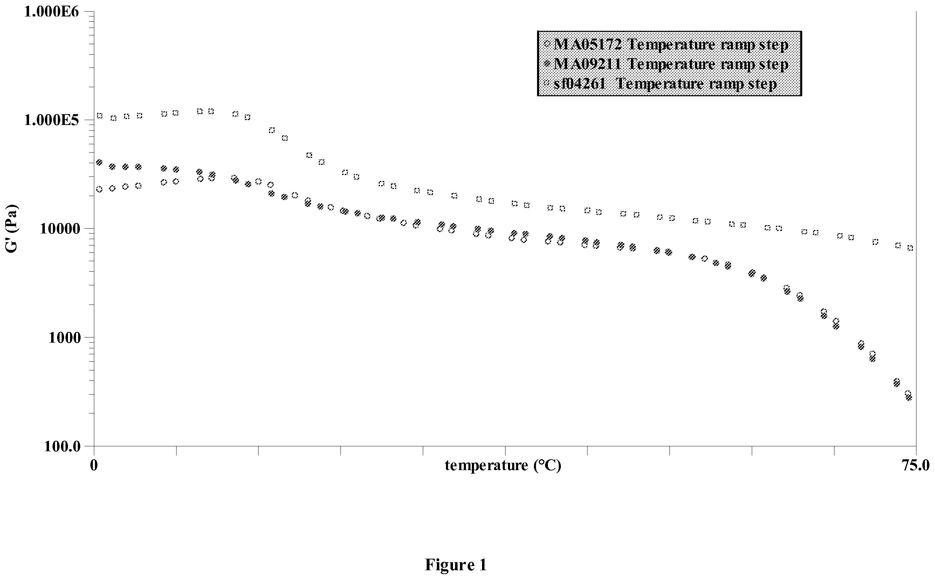

For wound dressings, the adhesive is often formulated to be soft and gentle, which is characterized by the low adhesive shear storage modulus G'[Pa]. The temperature ramp plot of shear modulus G'[Pa] vs. temperature for the adhesive compositions prepared with silica and without silica shows some differences in G' (see Figure 1 and Table 8).

**Table 8**

| | Shear Modulus G'[Pa] at Temperature | | | | | |
| --- | --- | --- | --- | --- | --- | --- |
| Adhesive | 10 °C | 20 °C | 30 °C | 40 °C | 50 °C | 60 °C |
| $SiO_2$ | 2.80E4 | 1.80E4 | 1.05E4 | 7740 | 6384 | 3835 |
| No $SiO_2$ | 1.18E5 | 4.67E4 | 2.22E4 | 1.63E4 | 1.32E4 | 1.07E4 |

The G' for the adhesive with silica is one order of magnitude lower than the adhesive without silica. Between 20 and 30°C, the decrease in % shear modulus for adhesive with $SiO_2$ was greater than that for the adhesive without silica. The low G' value for the adhesive with $SiO_2$ at ambient conditions indicates the soft characteristics of the adhesive, which is a critical requirement for adhesive to be used in low trauma application. The temperature at which $\tan \delta = 1$ was higher for the adhesive without $SiO_2$, (86.3 vs 63.3 °C) indicates the stiffness of adhesive. In addition, application of the adhesive without silica left behind a residue upon removal and was slightly aggressive.

b) Comparison of Loss Tangent ($\tan \delta$)

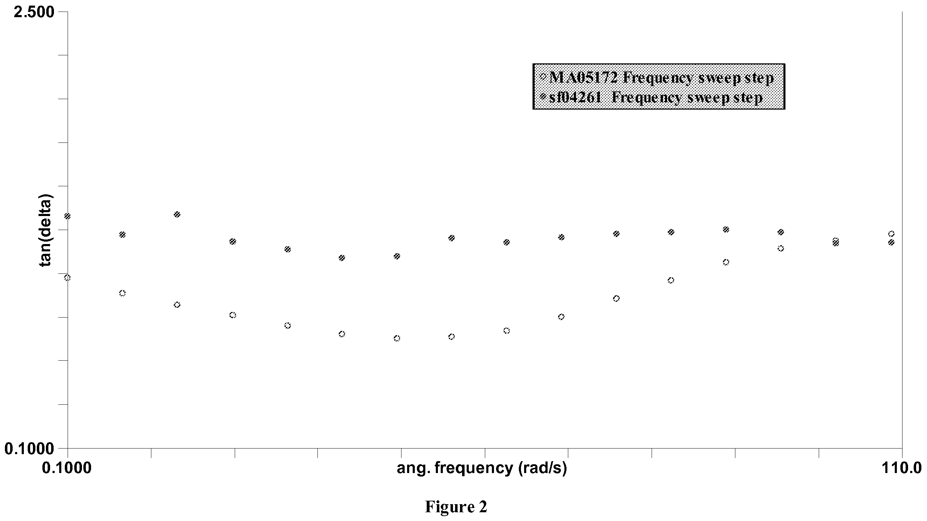

The frequency sweep plot of loss tangent ($\tan \delta$) vs. angular frequency was obtained at 37°C for adhesives prepared with and without silica. At 37°C, from 0.1 to 50.0 rad/sec, $\tan \delta$ was lower for the adhesive with $SiO_2$, indicating better viscous characteristics of the adhesive (see Figure 2).

c) Comparison of Viscosity

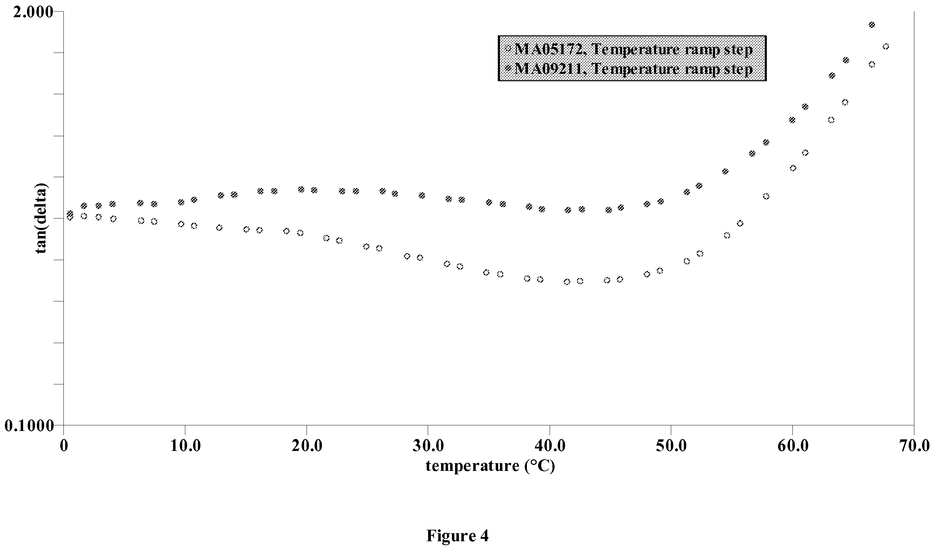

The frequency sweep plot of viscosity vs angular frequency was obtained at 37°C for adhesives prepared with and without silica. The viscosity at 37°C for the adhesive with $SiO_2$ was more linear as compared to the adhesive without $SiO_2$, indicating for the former adhesive that storage modulus (G') and loss modulus (G") are independent of frequency and the particles of silica and CMC are uniformly dispersed in the adhesive matrix (Figure 4). Hence, this demonstrates the significance of silica in the adhesive as a rheology modifier and softening agent in the designed application range.

**Claims**

1. An adhesive composition applicable to skin comprising:

   (i) a base polymer comprising at least one copolymer selected from the group consisting of styrene isoprene styrene, styrene butadiene styrene, styrene ethylene-butylene styrene, styrene-ethylene-styrene, styrene-propylene-styrene, and ethylene vinyl acetate;
   (ii) silica;
   (iii) coconut oil;
   (iv) a mineral oil;
   (v) a tackifier selected from the group consisting of natural rosin, modified rosin, glycerol ester of natural rosin, glycerol ester of modified rosin, pentaerythritol ester of natural rosin, pentaerythritol ester of modified rosin, phenolic-modified terpene resin, aliphatic petroleum hydrocarbon resin, and cycloaliphatic resin; and
   (vi) a gel-thickener selected from the group consisting of carboxymethylcellulose (CMC), hydroxyethylcellulose (HEC), hydroxypropylcellulose (HPC), methylcellulose, hydroxypropylmethylcellulose, sodium starch glycolate, polyvinyl alcohol, polyethylene glycol, pectin, carrageenan, and gelatin;
   wherein the composition is silicone-free; and
   wherein the ratio of

   (a) base polymer to coconut oil ranges from 1:1 to 1.5:1;
   (b) base polymer to mineral oil ranges from 0.5:1 to 0.6:1;
   (c) base polymer to tackifier ranges from 1.3:1 to 1.7:1;
   (d) base polymer to silica ranges from 38:1 to 78:1;
   (e) base polymer to gel-thickener ranges from 0.7:1 to 2.5:1;
   (f) mineral oil to coconut oil ranges from 2:1 to 2.5:1.

2. The adhesive composition of claim 1, wherein the silica compound is fumed silica.

3. The adhesive composition of claim 1, wherein the amount of the mineral oil is in the range of 22%-44.9% (w/w) of the total composition.

4. The adhesive composition of claim 1, wherein the amount of the gel-thickener is in the range of 1.8%-19.2% (w/w) of the total composition.

5. The adhesive composition of claim 1, wherein the amount of the base polymer is in the range of about 14%-28% (w/w) of the total composition, and wherein the amount of silica is in the range of 0.2%-9.4% (w/w) of the total composition.

6. The adhesive composition of claim 1, wherein the amount of the coconut oil is in the range of about 10%-21.5% (w/w) of the total composition.

7. A medical adhesive device comprising the adhesive of claim 1, wherein the amount of the coconut oil and mineral oil is in the range of about 48%-60% (w/w) of the total composition.

**Patentansprüche**

1. Haftzusammensetzung, anwendbar auf die Haut, umfassend:

   (i) ein Basispolymer, umfassend mindestens ein Copolymer, ausgewählt aus der Gruppe bestehend aus Styrol-Isopren-Styrol, Styrol-Butadien-Styrol, Styrol-Ethylen-Butylen-Styrol, Styrol-Ethylen-Styrol, Styrol-Propylen-Styrol und Ethylenvinylacetat;
   (ii) Siliciumdioxid;
   (iii) Kokosöl;
   (iv) ein Mineralöl;
   (v) einen Klebrigmacher, ausgewählt aus der Gruppe bestehend aus natürlichem Kolophonium, modifiziertem Kolophonium, Glycerinester von natürlichem Kolophonium, Glycerinester von modifiziertem Kolophonium, Pentaerythritester von natürlichem Kolophonium, Pentaerythritester von modifiziertem Kolophonium, phenolisch modifiziertem Terpenharz, aliphatischem Erdölkohlenwasserstoffharz und cycloaliphatischem Harz; und
   (vi) ein Gelverdickungsmittel, ausgewählt aus der Gruppe bestehend aus Carboxymethylcellulose (CMC), Hydroxyethylcellulose (HEC), Hydroxypropylcellulose (HPC), Methylcellulose, Hydroxypropylmethylcellulose, Natriumstärkeglycolat, Polyvinylalkohol, Polyethylenglycol, Pektin, Carrageen und Gelatine;
   wobei die Zusammensetzung silikonfrei ist; und
   wobei das Verhältnis von

   (a) Basispolymer zu Kokosnussöl im Bereich von 1:1 bis 1,5:1 liegt;
   (b) Basispolymer zu Mineralöl im Bereich von 0,5:1 bis 0,6:1 liegt;
   (c) Basispolymer zu Klebrigmacher im Bereich von 1,3:1 bis 1,7:1 liegt;
   (d) Basispolymer zu Siliciumdioxid im Bereich von 38:1 bis 78:1 liegt;
   (e) Basispolymer zu Gelverdickungsmittel im Bereich von 0,7:1 bis 2,5:1 liegt;
   (f) Mineralöl zu Kokosöl im Bereich von 2:1 bis 2,5:1 liegt.

2. Haftzusammensetzung nach Anspruch 1, wobei die Siliciumdioxidverbindung pyrogenes Siliciumdioxid ist.

3. Haftzusammensetzung nach Anspruch 1, wobei die Menge des Mineralöls im Bereich von 22 % - 44,9 % (Gew./Gew.) der Gesamtzusammensetzung liegt.

4. Haftzusammensetzung nach Anspruch 1, wobei die Menge des Gelverdickungsmittels im Bereich von 1,8 % - 19,2 % (Gew./Gew.) der Gesamtzusammensetzung liegt.

5. Haftzusammensetzung nach Anspruch 1, wobei die Menge des Basispolymers im Bereich von etwa 14 % - 28 % (Gew./Gew.) der Gesamtzusammensetzung liegt und wobei die Menge an Siliciumdioxid im Bereich von 0,2 % - 9,4 % (Gew./Gew.) der Gesamtzusammensetzung liegt.

6. Haftzusammensetzung nach Anspruch 1, wobei die Menge des Kokosöls im Bereich von etwa 10 % - 21,5 % (Gew./Gew.) der Gesamtzusammensetzung liegt.

7. Medizinische Haftvorrichtung, umfassend das Haftmittel nach Anspruch 1, wobei die Menge des Kokosöls und Mineralöls im Bereich von etwa 48 % - 60 % (Gew./Gew.) der Gesamtzusammensetzung liegt.

**Revendications**

1. Composition adhésive applicable sur la peau comprenant :

   (i) un polymère de base comprenant au moins un copolymère choisi dans le groupe constitué de styrène isoprène styrène, styrène butadiène styrène, styrène éthylène-butylène styrène, styrène-éthylène-styrène, styrène-propylène-styrène et éthylène-acétate de vinyle ;

(ii) de la silice ;

(iii) de l'huile de noix de coco ;

(iv) une huile minérale ;

(v) un agent poisseux choisi dans le groupe constitué de colophane naturelle, colophane modifiée, ester de glycérol de colophane naturelle, ester de glycérol de colophane modifiée, ester de pentaérythritol de colophane naturelle, ester de pentaérythritol de colophane modifiée, résine terpénique phénolique modifiée, résine d'hydrocarbure de pétrole aliphatique et résine cycloaliphatique ; et

vi) un épaississant de gel choisi dans le groupe constitué de carboxyméthylcellulose (CMC), hydroxyéthylcellulose (HEC), hydroxypropylcellulose (HPC), méthylcellulose, hydroxypropylméthylcellulose, glycolate sodique d'amidon, poly(alcool vinylique), polyéthylène glycol, pectine, carraghénane et gélatine ;

dans laquelle la composition est exempte de silicone ; et

dans lequel le rapport

(a) du polymère de base à l'huile de noix de coco varie de 1:1 à 1,5:1 ;

(b) du polymère de base à l'huile minérale varie de 0,5:1 à 0,6:1 ;

(c) du polymère de base à l'agent poisseux varie de 1,3:1 à 1,7:1 ;

(d) du polymère de base à la silice varie de 38:1 à 78:1 ;

(e) du polymère de base à l'épaississant du gel varie de 0,7:1 à 2,5:1 ;

(f) de l'huile minérale à l'huile de noix de coco varie de 2:1 à 2,5:1.

2. Composition adhésive selon la revendication 1, dans laquelle le composé de silice est de la silice pyrogénée.

3. Composition adhésive selon la revendication 1, dans laquelle la quantité d'huile minérale se situe dans la plage de 22 % à 44,9 % (p/p) de la composition totale.

4. Composition adhésive selon la revendication 1, dans laquelle la quantité d'épaississant de gel se situe dans la plage de 1,8 % à 19,2 % (p/p) de la composition totale.

5. Composition adhésive selon la revendication 1, dans laquelle la quantité de polymère de base se situe dans la plage d'environ 14 % à 28 % (p/p) de la composition totale, et dans laquelle la quantité de silice se situe dans la plage de 0,2 % à 9,4 % (p/p) de la composition totale.

6. Composition adhésive selon la revendication 1, dans laquelle la quantité d'huile de noix de coco se situe dans la plage d'environ 10 % à 21,5 % (p/p) de la composition totale.

7. Dispositif adhésif médical comprenant l'adhésif selon la revendication 1, dans lequel la quantité d'huile de noix de coco et d'huile minérale se situe dans la plage d'environ 48 % à 60 % (p/p) de la composition totale.

**Figure 1**

Figure 2

**Figure 3**

Figure 4

EP 2 970 729 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4505976 A